(19) Europäisches Patentamt
European Patent Office
Office européen des brevets

(11) Numéro de publication: **0 096 598**
**A1**

(12) # DEMANDE DE BREVET EUROPEEN

(21) Numéro de dépôt: 83400482.2

(22) Date de dépôt: 09.03.83

(51) Int. Cl.³: C 01 B 33/28
C 07 C 7/13, C 07 C 4/06
B 01 J 29/28

(30) Priorité: 01.04.82 FR 8205665

(43) Date de publication de la demande:
21.12.83 Bulletin 83/51

(84) Etats contractants désignés:
BE DE GB IT NL

(71) Demandeur: COMPAGNIE FRANCAISE DE RAFFINAGE
Société anonyme dite:
5, rue Michel-Ange
F-75781 Paris Cedex 16(FR)

(72) Inventeur: Bourgogne, Michel
19 Résidence Albert Schweitzer
F-68400 Riedisheim(FR)

(72) Inventeur: Guth, Jean-Louis
59, rue Bellevue
F-68200 Brunstatt(FR)

(72) Inventeur: Szabo, Georges
38, rue de Normandie
F-76250 Montivilliers(FR)

(72) Inventeur: Wey, Raymond
29, rue George Sand
F-68200 Mulhouse(FR)

(74) Mandataire: Jolly, Jean-Pierre et al,
Cabinet BROT et JOLLY 83, rue d'Amsterdam
F-75008 Paris(FR)

(54) **Procédé de préparation d'une zéolite, zéolite obtenue et applications.**

(57) L'invention concerne la préparation de la zéolite ZK 5, à partir d'une zéolite de départ choisie dans le groupe constitué par les zéolites P (Cl), P' (Cl), Q (Br) et Q' (Br).

Selon l'invention les ions baryum contenus dans la zéolite de départ sont extraits de celle-ci à l'aide d'un agent fixant le barym, choisi dans le groupe constitué par les agents précipitants formant avec les ions baryum un composé qui précipite et les agents complexants formant avec les ions baryum un complexe de baryum.

La zéolite ZK5 obtenue peut être appliquée à la séparation ou au craquage d'hydrocarbures.

EP 0 096 598 A1

Procédé de préparation de la zéolite ZK 5, zéolite ZK 5 obtenue par ce procédé et applications de cette zéolite.

La présente invention concerne un procédé de préparation de la zéolite ZK 5. L'invention concerne également des applications de cette zéolite, notamment à la séparation de composés tels que des hydrocarbures et comme catalyseur de craquage d'hydrocarbures.

Les zéolites constituent une famille d'aluminosilicates alcalins ou alcalinoterreux, comprenant plusieurs dizaines de composés qui existent à l'état naturel.

La structure des zéolites peut être définie comme un réseau tridimensionnel de tétraèdres de formule $Si\ O_4$ et $Al\ O_4$ reliés par des ponts oxygène. Ce réseau, dont la géométrie varie d'une zéolite à une autre, constitue un ensemble de cavités reliées par des ouvertures de formes et de dimensions variables, les caractéristiques de cet ensemble étant représentatives d'une zéolite donnée. Ces cavités sont occupées par des ions ou des molécules d'eau ayant une grande liberté de mouvement.

Les zéolites peuvent être différenciées les unes des autres par leur composition chimique et, plus spécialement, de façon précise, par leur spectre de diffraction des rayons X.

Leur spectre de diffraction de rayons X permet de déterminer les espacements <u>d</u> entre les plans réticulaires des cristaux, exprimés en Å, et les intensités relatives des raies du diagramme de diffraction.

Des modifications mineures quant aux espacements réticulaires et aux intensités observables sur certains diagrammes sont dues à des variations de composition chimique comme le remplacement de certains cations par d'autres ou à des variations du rapport $Si\ O_2/Al_2\ O_3$, mais elles ne signifient nullement une variation structurale importante de la zéolite.

La dimension déterminée des pores permet, pour une

zéolite donnée, d'adsorber ou de rejeter des molécules déterminées en fonction de leurs dimensions.

Les zéolites ont donc été utilisées pour la séparation de différents composés, par exemple pour le séchage ou la purification de gaz. Elles sont également utilisées comme catalyseurs ou supports de catalyseurs de conversion de composés, par exemple dans des réactions de craquage, d'alkylation ou d'isomérisation d'hydrocarbures.

Les propriétés intéressantes de ces zéolites ont conduit à la recherche de procédés de fabrication de zéolites synthétiques. De nombreuses zéolites ont été synthétisées et font l'objet de multiples brevets.

Ces zéolites synthétiques sont communément désignées par un sigle, comme les zéolites A, X, Y, W, ZSM 11, ZSM 12, etc...

Une zéolite particulière, la ZK 5, a ainsi été préparée par un procédé décrit dans l'ouvrage de D.W. BRECK "Zeolite Molecular Sieves", pages 309 et 310.

Cette méthode consiste à partir d'un gel d'alumino-silicate auquel on ajoute un cation azoté dibasique de formule :

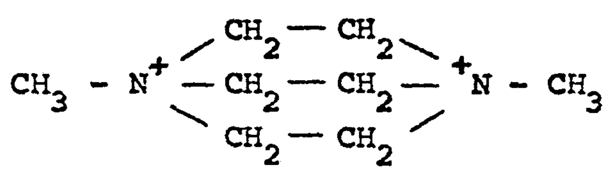

Ce cation est introduit sous la forme d'un silicate de 1,4-diméthyl 1-1,4-diazoniacyclo (2,2,2,) octane.

La composition du mélange réactionnel a les rapports molaires suivants :

- $SiO_2/Al_2O_3$ : 4 à 11,
- $Na_2O/Al_2O_3 + C_8H_{18}N_2O/Al_2O_3$ : 6 à 19,
- $Na_2O/Al_2O_3$ : 1,5 à 2,3,
- $H_2O/Al_2O_3$ : 200 à 700.

L'alumine est ajoutée sous la forme d'une solution d'aluminate de sodium, qui est ensuite mélangée avec la solution de silicate d'ammonium quaternaire pour

former un gel amorphe. La ZK 5 est obtenue par chauffage du mélange réactionnel à 100°C pendant 9 jours. La composition chimique de la zéolite obtenue est :

$$0,3\text{-}0,7\ Na_2O/0,3\text{-}0,7\ RO/Al_2O_3/4,0\text{-}6,0\ SiO_2/6\text{-}10\ H_2O$$

où

$$R = C_{18}\ H_{18}\ N_2^{2+}$$

Cette méthode a l'inconvénient de nécessiter l'emploi d'un produit cher, à savoir le silicate de 1,4-diméthyl 1-1,4-diazoniacyclo (2,2,2,) octane.

Une autre méthode consiste à préparer une des phases intermédiaires appelées P(Cl), P'(Cl), Q(Br) ou Q'(Br). Ces phases intermédiaires sont obtenues par réaction d'un halogénure de baryum sur un gel d'alumino-silicate de baryum ou de potassium (voir brevet US n° 2 413 134) ou sur certaines zéolites comme l'analcite (voir également brevet US n° 2 413 134), la zéolite Y, la zéolite X ou la chabazite (voir l'article de R.M BARRER et C. MARCILLY "Synthesis and Nature of some Salt-bearing Aluminosilicates", paru dans The Journal of the Chemical Society, section A, Inorganic, Physical and Theorical Chemistry, pages 2735 à 2745 (1970).

Ce brevet et cet article donnent comme conditions opératoires des températures d'au moins 100°C, pouvant atteindre 400°C et des durées de réaction de plusieurs jours.

Si l'halogénure de baryum est le chlorure de baryum, on obtient la zéolite P (Cl) ou P' (Cl). Si l'halogénure de baryum est le bromure de baryum, on obtient la zéolite Q (Br) ou Q' (Br).

Les zéolites P (Cl) ou Q (Br) sont obtenues à partir de l'analcite ou de la zéolite Y, les zéolites P' (Cl) ou Q' (Br) à partir de la zéolite X.

Ces zéolites contiennent du baryum comme cation de compensation et, sous forme de sel d'inclusion, l'halogénure

de baryum.

L'halogénure est ensuite extrait de la zéolite soit par lavage à l'eau, comme décrit dans le brevet US n° 2 413 134, soit par lavage à l'aide de solutions de nitrate (de sodium par exemple), comme décrit dans l'article de BARRER et MARCILLY précité. On obtient ainsi une zéolite qui a la structure de la zéolite ZK 5.

Ces méthodes ont toutefois l'inconvénient d'être longues et, étant donné les conditions sévères des lavages, conduisent souvent à une recristallisation de la zéolite en d'autres phases (voir à ce sujet le tableau 6, page 2739, de l'article de BARRER et MARCILLY).

La Demanderesse a conçu un procédé plus simple de préparation de la zéolite ZK 5.

L'invention a par conséquent pour objet un procédé de préparation de la zéolite ZK 5, à partir d'une zéolite de départ choisie dans le groupe constitué par les zéolites P (Cl), P' (Cl), Q (Br) et Q' (Br), ladite zéolite de départ étant préparée de façon connue en soi, ce procédé étant caractérisé en ce que les ions baryum contenus dans la zéolite de départ sont extraits de celle-ci à l'aide d'un agent fixant le baryum, choisi dans le groupe constitué par les agents précipitants formant avec les ions baryum un composé qui précipite et les agents complexants formant avec les ions baryum un complexe du baryum.

Dans la forme de mise en oeuvre du procédé selon l'invention utilisant un agent précipitant, celui-ci est choisi parmi les composés solubles aptes à former avec le baryum un composé insoluble aisément séparable de la zéolite ZK 5 et sans altération de la structure de cette zéolite. Parmi les agents envisageables, on peut citer les rhodizonates, les oxalates et les carbonates des métaux alcalins ou d'ammonium. Dans le cas où l'on utilise un carbonate alcalin ou d'ammonium,

il se forme un précipité de carbonate de baryum qui est aisément éliminé de la zéolite par dissolution à pH compris entre 1 et 5,5 à l'aide de solutions du type mélanges tampons acétiques, acide chlorhydrique dilué, etc...

Si l'on utilise un agent complexant, celui-ci est choisi parmi les composés solubles, tels que les sels de l'acide éthylènediamine tétraacétique (EDTA) ou de l'acide pentaacétique de la diéthylènetriamine (DTPA), aptes à former avec les ions baryum des complexes solubles. Dans ce cas, la zéolite ZK 5 est obtenue par simple filtration de la solution contenant les complexes fournis avec le baryum.

Dans le procédé selon l'invention, on peut donc partir, en particulier, d'une zéolite P (Cl) ou P' (Cl).

Les diagrammes de diffraction de rayons X des zéolites P (Cl) et P' (Cl) sont donnés dans le Tableau I ci-après où <u>d</u> désigne l'espacement entre deux plans réticulaires, exprimé en Å, et $I/I_0$ l'intensité relative des raies du diagramme.

TABLEAU I

| P (Cl) | | P' (Cl) | |
|---|---|---|---|
| d(Å) | $I/I_0$ | d(Å) | $I/I_0$ |
| 13,20 | 100 | - | - |
| 9,36 | 15 | - | - |
| - | - | - | - |
| - | - | - | - |
| - | - | 5,93 | 19 |
| - | - | - | - |
| 4,99 | 22 | 5,01 | 60 |
| 4,64 | 11 | - | - |
| 4,39 | 77 | 4,43 | 80 |
| 4,17 | 36 | 4,20 | 57 |

TABLEAU I (suite)

| P (Cl) | | P' (Cl) | |
|---|---|---|---|
| d(Å) | $I/I_o$ | d(Å) | $I/I_o$ |
| 3,96 | 9 | - | - |
| 3,80 | 47 | 3,84 | 40 |
| 3,66 | 20 | - | - |
| - | - | - | - |
| 3,29 | 15 | 3,32 | 27 |
| 3,19 | 63 | 3,22 | 38 |
| 3,11 | 14 | 3,13 | 30 |
| 3,03 | 94 | 3,05 | 100 |
| 2,95 | 6 | - | - |
| 2,87 | 18 | 2,90 | 22 |
| 2,81 | 83 | 2,84 | 57 |
| 2,74 | 6 | 2,77 | 26 |
| 2,69 | 6 | 2,71 | 17 |
| 2,63 | 26 | 2,66 | 17 |
| 2,58 | 13 | 2,60 | 26 |
| 2,53 | 35 | 2,56 | 65 |

Les conditions opératoires utilisables dans le procédé selon l'invention pour extraire $BaCl_2$ de la zéolite P (Cl) ou de la zéolite P' (Cl) sont données dans le Tableau II ci-après pour différents agents d'extraction.

**TABLEAU II**

| Agent d'extraction | Paramètres | | | | | | | | | |
|---|---|---|---|---|---|---|---|---|---|---|
| | A | | B | | C | | D | | E | |
| | D.L. | D.P. | D.L. | D.P. | D.L. | D.P. | D.L. | D.P. | D.L. | D.P. |
| Carbonates alcalins ou d'ammonium | M/10-2M | M/5-1,5M | M/10-2M | M/4-M | 2-25 | 5-20 | 20-280 | 80-200 | 0,5-192 | 1-120 |
| Sels de l'acide EDTA | M/10-2M | M/5-M | M/10-2M | M/5-1,5M | 2-25 | 5-20 | 40-180 | 60-150 | 6-192 | 12-96 |
| Sels de l'acide DTPA | M/10-2M | M/5-M | M/2-2M | M-1,5M | 2-25 | 5-20 | 20-125 | 40-110 | 6-144 | 12-72 |

A : concentration de l'agent fixant le baryum (en mole/l),
B : concentration (en mole/l) d'une base, telle que la soude, qui peut être employée en compagnie de l'agent d'extraction,
C : rapport liquide/solide en poids,
D : température en °C,
E : durée du traitement (en heures),
D.L. : domaine large,
D.P. : domaine préféré.

Dans le cas où l'on utilise un carbonate alcalin ou d'ammonium pour extraire les ions baryum, le précipité de carbonate de baryum est dissous à un pH compris entre 1 et 5,5, sans altération de la structure de la zéolite, à l'aide de solutions comme les tampons acétiques $CH_3COOH/CH_3COONa$ et $CH_3COOH/CH_3COONH_4$ , l'acide chlorhydrique dilué, etc...

Les conditions opératoires de dissolution du carbonate de baryum à pH compris entre 1 et 5,5 sont données pour les cas indiqués ci-dessus dans le Tableau III ci-après.

TABLEAU III

| Agents dissolvant le carbonate de baryum | Paramètres | | | | | |
|---|---|---|---|---|---|---|
| | A | B | | C | | D |
| | D.L. | D.L. | D.P. | D.L. | D.P. | D.L. |
| Tampons acétiques | 0,5M-2M | 5-30 | 8-25 | 10-100 | 15-60 | 5-20 |
| Acide chlorhydrique | M/100-M/4 | 5-30 | 8-25 | 10-25 | | 5-20 |

A : concentration de l'agent dissolvant (en mole/l),
B : rapport liquide/solide en poids,
C : température en °C,
D : durée du traitement (en minutes),
D.L. : domaine large,
D.P. : domaine préféré.

La formule générale de la zéolite ZK 5 préparée par le procédé selon l'invention est :

$$0,5-0,9M_2O/0,2-0,5BaO/Al_2O_3/2,5-7SiO_2/0-0,3BaCl_2/0-7H_2O,$$

où M est un cation alcalin.

Son diagramme de diffraction de rayons X est donné dans le Tableau IV ci-après.

0096598

TABLEAU IV

| d(Å) | $I/I_o$ |
|---|---|
| 13,26 | 50 |
| 9,34 | 100 |
| 7,65 | 12 |
| 6,60 | 15 |
| 5,92 | 22 |
| 5,40 | 34 |
| 5,01 | 15 |
| 4,67 | 9 |
| 4,42 | 56 |
| 4,19 | 50 |
| 3,99 | 26 |
| 3,83 | 40 |
| 3,67 | 15 |
| 3,42 | 19 |
| 3,30 | 9 |
| 3,21 | 44 |
| 3,12 | 11 |
| 3,05 | 74 |
| 2,96 | 23 |
| 2,89 | 9 |
| 2,83 | 53 |
| 2,77 | 19 |
| 2,71 | 11 |
| 2,65 | 22 |
| 2,60 | 7 |
| 2,55 | 22 |

L'invention permet d'obtenir de la zéolite ZK 5 en poudre ou conditionnée à partir de zéolites P (Cl), P' (Cl), Q (Br) ou Q' (Br) en poudre ou déjà conditionnées, c'est-à-dire agglomérées (pastilles, granulés, extrudés, etc...) sans liant. Cette mise en forme des zéolites utilise des procédés connus, la pression mise en oeuvre ne devant pas excéder 10 tonnes/$cm^2$. L'agglomération de la poudre peut s'effectuer éventuellement en présence d'eau. Dans ce cas, la masse d'eau ne doit pas dépasser 50 % de la masse de zéolite engagée.

La zéolite ZK 5, préparée par le procédé selon l'invention, peut être notamment utilisée pour la séparation d'hydrocarbures aliphatiques saturés linéaires, d'hydrocarbures aliphatiques saturés ramifiés, d'hydrocarbures cycliques saturés ou d'hydrocarbures aromatiques.

Cette zéolite peut servir encore comme catalyseur de craquage d'hydrocarbures.

Les exemples qui suivent sont destinés à illustrer l'invention de façon non limitative.

L'Exemple 1 concerne la préparation de zéolites P (Cl) ou P' (Cl).

L'Exemple 2 concerne la préparation de zéolites ZK 5 à partir de zéolites P (Cl) ou P' (Cl) préparées selon l'Exemple 1.

L'Exemple 3 concerne des essais d'adsorption de différents hydrocarbures, effectués avec les zéolites ZK 5 préparées selon l'Exemple 2.

L'Exemple 4 concerne des essais de craquage d'hydrocarbures à l'aide d'une zéolite préparée selon l'Exemple 2.

## Exemple 1

Cet exemple concerne la préparation de zéolites P (Cl) ou P' (Cl).

### Préparation n° 1

On fait réagir dans un flacon de 500 ml en polytétrafluoroéthylène :

- 20 g de faujasite Na-Y en poudre ayant un rapport Si $O_2/Al_2O_3$ de 4,2,
- 3,5 g de silice amorphe en poudre contenant 16 % en poids d'eau,
- 240 g de chlorure de baryum Ba $Cl_2$, 2 $H_2O$,
- 240 ml d'eau.

Le Rapport global Si $O_2/Al_2$ $O_3$ du mélange réactionnel est égal à 5,5.

Le flacon en polytétrafluoroéthylène est placé dans un autoclave, qui est porté à la température de 250°C pendant 100 heures. Le mélange réactionnel est ensuite filtré et on sèche le solide obtenu à 60°C.

On obtient ainsi 28,7 g d'un produit qui est identifié comme étant une zéolite P (Cl) par son spectre de diffraction des rayons X.

Grâce à l'analyse du produit, on détermine que celui-ci a la formule suivante :

$1,18\ BaO/0,02\ Na_2O/Al_2O_3/6,48\ SiO_2/0,89\ BaCl_2/3,4\ H_2O$.

Préparation n° 2

On fait réagir, dans un flacon d'un litre en polytétrafluoroéthylène :

- 50 g de faujasite Na-Y sous forme d'extrudés, ayant un rapport Si $O_2/Al_2$ $O_3$ de 4,98,
- 600 g de chlorure de baryum Ba $Cl_2$, 2 $H_2O$,
- 600 ml d'eau.

Le flacon en polytétrafluoroéthylène est placé dans un autoclave, qui est porté à la température de 250°C pendant 120 heures. Le mélange réactionnel est ensuite filtré et on sèche le solide obtenu à 60°C.

On obtient ainsi 71 g d'un produit, qui est identifié comme étant une zéolite P (Cl) par son spectre de diffraction de rayons X. Sa formule est la suivante :

$1,18\ BaO/0,02\ Na_2O/Al_2O_3/5,91\ Si\ O_2/0,86\ Ba\ Cl_2/3,46H_2O$.

Préparation n° 3

L'expérience précédente est reconduite en utilisant cette fois-ci une faujasite Na-X sous forme d'extrudés,

ayant un rapport Si $O_2/Al_2O_3$ de 2,5.

On obtient, après transformation, filtration, et séchage à 60°C, 72 g de zéolite P' (Cl), identifiée par son spectre de diffraction des rayons X, de formule :

1,18 Ba $O/Al_2O_3$/3,2 Si $O_2$/0,7 Ba $Cl_2/H_2O$.

La valeur élevée du rapport Si $O_2/Al_2O_3$ des zéolites obtenues dans les trois préparations de cet exemple s'explique à la fois par une extraction partielle de l'aluminium au cours de la transformation et par la présence d'un peu de silice résiduelle amorphe dans le produit final lors de la transformation en présence de silice.

## Exemple 2

Cet exemple concerne la préparation de zéolites ZK 5 à partir de zéolites P (Cl) ou P' (Cl) préparées selon l'Exemple 1.

Préparation n° 1

On traite 1 g de zéolite P (Cl), préparée selon la préparation n° 1 de l'Exemple 1, par une solution aqueuse de carbonate de sodium contenant une mole par litre de carbonate de sodium. Le rapport liquide/solide, en poids, est, lors de ce traitement, égal à 10. Le traitement est effectué à 110°C pendant 72 heures. Il se forme du carbonate de baryum qui précipite. On recueille par filtration un solide qui est ensuite séché à 60°C.

Le carbonate de baryum est séparé de la zéolite par dissolution à l'aide d'une solution contenant, par litre, une mole d'acétate de sodium pour une mole d'acide acétique. Cette dissolution est effectuée à environ 20°C, par agitation du mélange pendant environ 10 minutes, le rapport liquide/solide, en poids, étant de 20.

On recueille par filtration 690 mg d'un solide, qui est séché à 60°C. Ce solide présente un spectre de diffraction des rayons X correspondant à une zéolite ZK 5, de formule :

$0,65\ Na_2O/0,41\ BaO/Al_2O_3/5,57\ SiO_2/0,1\ BaCl_2/6,73\ H_2O$.

Préparation n° 2

On traite 10 g de zéolite P (Cl), préparée selon la préparation n° 1 de l'Exemple 1, par une solution aqueuse de carbonate de sodium contenant une demi-mole par litre de carbonate de sodium. Le rapport liquide/solide, en poids, est, lors de ce traitement, égal à 8. Le traitement est effectué à 150°C pendant 60 heures. Après filtration et séchage du solide obtenu, la dissolution du carbonate de baryum est effectuée à l'aide d'une solution contenant, par litre, une mole d'acétate de sodium pour une mole d'acide acétique. La dissolution est effectuée dans les mêmes conditions que dans la préparation n° 1, le rapport liquide/solide, en poids, étant de 10.

On obtient 6,3 g de zéolite ZK 5, identifiée par son spectre de rayons X, de formule :

$0,54\ BaO/0,53\ Na_2O/Al_2O_3/5,66\ SiO_2/0,04\ BaCl_2/6,78\ H_2O$

Préparation n° 3

On traite 5 g de zéolite P (Cl), préparée selon la préparation n° 1 de l'Exemple 1, par une solution aqueuse de carbonate de sodium contenant une demi-mole par litre de carbonate de sodium. Le rapport liquide/solide, en poids, est, lors de ce traitement, égal à 15. Le traitement est effectué à 250°C pendant 2 heures. Après filtration et séchage du solide obtenu à 60°C, la dissolution du carbonate de baryum est effectuée à l'aide d'une solution décimolaire d'acide chlorhydrique, à 20°C environ, par agitation du mélange pendant environ 10 minutes, le rapport liquide/solide, en poids, étant égal à 15.

On obtient 3,4 g de zéolite ZK 5, identifiée par son spectre de diffraction de rayons X, de formule :

$0,59BaO/0,49\ Na_2O/Al_2O_3/5,79\ SiO_2/0,1\ BaCl_2/6,53\ H_2O$.

Préparation n° 4

On traite 50 g d'une zéolite P (Cl), préparée selon un mode opératoire analogue à la préparation n° 1 de

l'Exemple 1, par une solution aqueuse contenant par litre une demi-mole de carbonate d'ammonium et une demi-mole de soude, le rapport liquide/solide, en poids, étant égal à 10. Le traitement est effectué à 100°C, pendant 72 heures. Après filtration et séchage du solide obtenu, la dissolution du carbonate de baryum est effectuée à l'aide d'une solution contenant, par litre, une mole d'acétate d'ammonium pour une mole d'acide acétique. La dissolution est effectuée à 20°C environ, par agitation du mélange pendant environ 10 mn, le rapport liquide/solide, en poids, étant de 20. On obtient 32,5 g de zéolite ZK 5, identifiée par son spectre de rayons X, de formule :

$$0,59\ Na_2O/0,48\ BaO/Al_2O_3/5,17\ SiO_2/0,06\ BaCl_2/5,62\ H_2O.$$

Préparation n° 5

On traite 50 g de zéolite P (Cl), préparée selon la préparation n° 2 de l'Exemple 1, par une solution aqueuse du sel disodique de l'acide éthylènediamine-tétraacétique, contenant, par litre, un quart de mole dudit sel et une demi-mole de soude.

Le rapport liquide/solide, en poids, est égal à 10. Le traitement est effectué à 110°C pendant 72 heures. Le baryum, en présence de l'anion éthylènediamine-tétraacétique $[N_2(CH_2)_2(CO_2\ CH_2)_4]^{2-}H_2$ et en milieu basique, est complexé selon la réaction :

$$Ba^{2+} + [N_2(CH_2)_2(CO_2\ CH_2)_4]^{2-}H_2 \rightarrow Ba[N_2(CH_2)_2(CO_2CH_2)_4]^{2-} + 2H^+$$

Après filtration et séchage à 60°C, on obtient 35,6 g d'un solide présentant le spectre aux rayons X d'une zéolite ZK 5 et ayant pour formule :

$$0,79\ Na_2O/0,22\ BaO/Al_2O_3/5,67\ SiO_2/0,07\ BaCl_2/5,70\ H_2O.$$

Préparation n° 6

On traite 20 g de zéolite P (Cl), préparée selon la préparation n° 2 de l'Exemple 1, par une solution aqueuse du sel disodique de l'acide EDTA, contenant, par litre, un quart de mole dudit sel et une demi-mole de soude.

Le rapport liquide/solide, en poids, est égal à 8. Le traitement est effectué à 80°C pendant 60 heures. Après filtration et séchage à 60°C, on obtient 15,6 g de zéolite ZK 5, identifiée par son spectre de rayons X, de formule :

$0,67\ Na_2O/0,43\ BaO/Al_2O_3/5,62\ SiO_2/5,62\ H_2O$.

Préparation n° 7

On traite 3 g de zéolite P (Cl), préparée selon la préparation n° 2 de l'Exemple 1, par une solution du sel disodique de l'acide EDTA, contenant, par litre, un quart de mole dudit sel et une demi-mole de soude. Le rapport liquide/solide, en poids, est égal à 10. Le traitement est effectué à 125°C pendant 24 heures. Après filtration et séchage à 60°C, on obtient 2,1 g de zéolite ZK 5, identifiée par son spectre de rayons X, de formule :

$0,74\ Na_2O/0,37\ BaO/Al_2O_3/5,40\ SiO_2/5,61\ H_2O$.

Préparation n° 8

On traite 100 g d'une zéolite P (Cl), préparée selon un mode opératoire analogue à la préparation n° 1 de l'Exemple 1, par une solution aqueuse du sel disodique de l'acide EDTA contenant, par litre, une demi-mole dudit sel et une mole de soude. Le rapport liquide/solide, en poids, est égal à 10. Le traitement est effectué à 100°C pendant 72 heures.

Après filtration et séchage à 60°C, on obtient 60 g de zéolite ZK 5, identifiée par son spectre de rayons X, de formule :

$0,94\ Na_2O/0,17\ BaO/Al_2O_3/5,15\ SiO_2/0,2\ BaCl_2/5,85\ H_2O$.

Préparation n° 9

On traite 20 g d'une zéolite P (Cl), préparée selon un mode opératoire analogue à la préparation n° 1 de l'Exemple 1, par une solution composée d'un solvant constitué par un mélange à 50 % en volume d'eau et d'alcool éthylique et contenant, par litre, une demi-mole de sel disodique de l'acide EDTA et une mole de soude. Le rapport liquide/solide, en poids, est, lors de ce

traitement, égal à 15. Le traitement est effectué à 80°C pendant 72 heures.

Après filtration et séchage à 60°C, on obtient 10 g de zéolite ZK 5, identifiée par son spectre de rayons X, de formule :

$1,01\ Na_2O/0,18\ BaO/Al_2O_3/4,92\ SiO_2/0,3\ BaCl_2/5,64\ H_2O$.

<u>Préparation n° 10</u>

On traite 20 g de zéolite P (Cl), préparée selon la préparation n° 2 de l'Exemple 1, par une solution de l'acide pentaacétique de la diéthylène triamine (DTPA) contenant, par litre, un quart de mole dudit acide et 1,25 mole de soude. Le rapport liquide/solide, en poids, est égal à 10. Le traitement est effectué à 60°C pendant 90 heures. Après filtration et séchage à 60°C, on obtient 12,4 g de zéolite ZK 5, identifiée par son spectre de rayons X, de formule :

$0,81\ Na_2O/0,27\ BaO/Al_2O_3/5,62\ SiO_2/0,14\ BaCl_2/5,65\ H_2O$.

<u>Préparation n° 11</u>

On traite 50 g de zéolite P (Cl), préparée selon la préparation n° 2 de l'Exemple 1, par une solution de l'acide DTPA contenant, par litre, un quart de mole dudit acide et 1,25 mole de soude. Le rapport liquide/solide, en poids, est égal à 10. Le traitement est effectué à 85°C pendant 72 heures. Après filtration et séchage à 60°C, on obtient 35 g de zéolite ZK 5, identifiée par son spectre de rayons X, de formule :

$0,83\ Na_2O/0,25\ BaO/Al_2O_3/5,69\ SiO_2/0,09\ BaCl_2/5,52\ H_2O$.

<u>Préparation n° 12</u>

On traite 100 g de zéolite P (Cl), préparée selon la préparation n° 2 de l'Exemple 1, par une solution de l'acide DTPA, contenant, par litre, un quart de mole dudit acide et 1,25 mole de soude. Le rapport liquide/solide, en poids, est égal à 10. Le traitement est effectué à 100°C pendant 48 heures.

Après filtration et séchage à 60°C, on obtient 71 g

de zéolite ZK 5, identifiée par son spectre de rayons X, de formule :

$$0,82\ Na_2O/0,26\ BaO/Al_2O_3/5,55\ SiO_2/0,04\ BaCl_2/5,63\ H_2O.$$

Préparation n° 13

On traite 50 g de zéolite P' (Cl), préparée selon la préparation n° 3 de l'Exemple 1, par une solution aqueuse du sel disodique de l'acide éthylènediamine-tétraacétique, contenant, par litre, un quart de mole dudit sel et une demi-mole de soude. Le rapport liquide/solide, en poids, est égal à 10. Le traitement est effectué à 110°C, pendant 72 heures. Après filtration et séchage, on obtient 30 g de zéolite ZK 5, identifiée par son spectre de rayons X, de formule :

$$0,80\ Na_2O/0,21\ BaO/Al_2O_3/3\ SiO_2/0,05\ BaCl_2/7,3\ H_2O.$$

Exemple 3

Cet exemple concerne des essais d'adsorption de différents hydrocarbures effectués avec les zéolites ZK 5 préparées selon l'Exemple 2.

Essais A

Les mesures d'adsorption ont été réalisées par méthode gravimétrique, en utilisant une balance du type MAC BAIN à spirales en verre de silice.

Les échantillons de zéolite ZK 5, après dégazage et déshydratation par chauffage à 350°C, sous une pression réduite de $10^{-2}$ torr, pendant 2 heures, sont mis en contact avec un hydrocarbure à l'état gazeux, du normal-hexane ou du cyclohexane. L'hydrocarbure se trouve à la tension de vapeur de l'hydrocarbure considéré à 13 °C, les vapeurs d'hydrocarbure provenant d'une source maintenue à 13°C.

Lorsque la température de la zéolite, qui est donc initialement à 350°C, est descendue à 20°C, la mesure est effectuée.

Les résultats des essais figurent dans le Tableau V ci-après.

TABLEAU V

| Echantillon de zéolite ZK 5 correspondant à la préparation n° | Grammes de n-hexane adsorbé pour 100 g d'échantillon anhydre | Grammes de cyclohexane adsorbé pour 100 g d'échantillon anhydre |
|---|---|---|
| 1 | 11,85 | non mesuré |
| 2 | 12,98 | 1,35 |
| 3 | 10,20 | non mesuré |
| 4 | 11,95 | - |
| 5 | 10,87 | - |
| 6 | 8,43 | - |
| 7 | 9,96 | - |
| 8 | 13,94 | 0,75 |
| 10 | 9,86 | non mesuré |
| 11 | 11,00 | - |
| 12 | 10,86 | 1,25 |

L'examen de ce Tableau montre que la zéolite ZK 5 adsorbe beaucoup mieux le normal-hexane que le cyclohexane et permet donc de séparer ces deux hydrocarbures.

Essais B

Ces essais d'adsorption ont été effectués avec une zéolite ZK 5 préparée selon un mode opératoire analogue à la préparation n° 4 de l'Exemple 2, laquelle zéolite ZK 5 est issue d'une zéolite P (Cl) préparée selon un mode opératoire analogue à la préparation n° 1 de l'Exemple 1.

L'échantillon a été dégazé et déshydraté par chauffage à 300°C, sous une pression réduite de $10^{-3}$ torr, pendant 18 heures. Après que sa température soit descendue à 20°C, l'échantillon est mis en contact pendant 4 heures avec différents hydrocarbures à l'état gazeux.

L'hydrocarbure se trouve à la tension de vapeur de l'hydrocarbure considéré à 0°C, les vapeurs d'hydrocarbure provenant d'une source maintenue à 0°C.

Par pesée de l'échantillon avant et après chaque adsorption, on peut ainsi connaître les pourcentages en poids d'hydrocarbure adsorbé qui sont, respectivement, pour :

- le normal-hexane : 7,83 %,
- le 3-méthyl pentane : 0 %,
- le benzène : 0,67 %,
- le cyclohexane : 0,10 % .

Ces essais montrent que l'on peut séparer grâce à la zéolite ZK 5 différents hydrocarbures ayant le même nombre d'atomes de carbone.

Exemple 4

Cet exemple concerne des essais de craquage d'hydrocarbures à l'aide d'une zéolite ZK 5 identique à celle utilisée dans les essais B de l'Exemple 3.

Préalablement aux essais de craquage proprement dits, la zéolite ZK 5, qui se trouve sous forme sodique, subit un traitement pour la transformer en forme acide, c'est-à-dire pour remplacer les ions sodium par des ions hydrogène.

La zéolite est traitée trois fois par une solution de chlorure d'ammonium à une température de 80°C pendant deux heures. Elle est essorée après chaque traitement, puis calcinée à 550°C.

La zéolite ZK 5-H ainsi obtenue est utilisée pour craquer à différentes températures une charge constituée par un mélange contenant 50 % de normal-hexane et 50 % de 3-méthyl pentane. Par analyse par chromatographie des gaz sortant du réacteur de conversion, on peut connaître la conversion de normal-hexane et du 3-méthyl pentane.

On a obtenu les résultats figurant dans le Tableau VI ci-après.

TABLEAU VI

| Température en °C | Conversion en % du : | |
| --- | --- | --- |
| | normal-hexane | 3-méthyl pentane |
| 290 | 17,01 | 0 |
| 320 | 19,86 | 0 |
| 360 | 20,75 | 0 |
| 410 | 28,83 | 0 |
| 450 | 30,45 | 0 |

La zéolite ZK 5 préparée par le procédé selon l'invention est donc un bon catalyseur sélectif de craquage des hydrocarbures.

REVENDICATIONS

1.- Procédé de préparation de la zéolite ZK 5, à partir d'une zéolite de départ choisie dans le groupe constitué par les zéolites P (Cl), P' (Cl), Q (Br) et Q' (Br), ladite zéolite de départ étant préparée de façon connue en soi, ce procédé étant caractérisé en ce que les ions baryum contenus dans la zéolite de départ sont extraits de celle-ci à l'aide d'un agent fixant le baryum, choisi dans le groupe constitué par les agents précipitants formant avec les ions baryum un composé qui précipite et les agents complexants formant avec les ions baryum un complexe du baryum.

2.- Procédé selon la revendication 1, caractérisé en ce que l'agent précipitant est choisi dans le groupe constitué par les rhodizonates, les oxalates, les carbonates des métaux alcalins et le carbonate d'ammonium.

3.- Procédé selon la revendication 2, caractérisé en ce que l'agent précipitant est un carbonate choisi dans le groupe constitué par le carbonate d'ammonium et les carbonates d'un métal alcalin et en ce que le carbonate de baryum formé est dissous à un pH compris entre 1 et 5,5, à l'aide d'une solution choisie dans le groupe constitué par les mélanges tampons acétiques et l'acide chlorhydrique dilué.

4.- Procédé selon la revendication 1, caractérisé en ce que l'agent complexant est choisi dans le groupe constitué parmi les composés solubles aptes à former avec les ions baryum des complexes solubles séparables de la zéolite ZK 5 par filtration.

5.- Procédé selon la revendication 4, caractérisé en ce que l'agent complexant est choisi dans le groupe constitué par les sels de l'acide éthylène diamine tétraacétique et de l'acide diéthylènetriamine pentaacétique.

6.- Procédé selon l'une des revendications 3 et 5,

caractérisé en ce que, lors de l'extraction, le rapport liquide/solide, en poids, est compris entre 2 et 25 et, de préférence, entre 5 et 20.

7.- Procédé selon l'une des revendications 3 et 6, caractérisé en ce que, lors de l'extraction, la concentration de l'agent précipitant est comprise, en mole par litre, entre $\frac{M}{10}$ et 2 M et, de préférence, entre $\frac{M}{5}$ et 1,5 M.

8.- Procédé selon l'une des revendications 3, 6 et 7, caractérisé en ce qu'une base est employée conjointement avec l'agent d'extraction et en ce que la concentration de ladite base est comprise, en mole par litre, entre $\frac{M}{10}$ et 2 M et de préférence entre $\frac{M}{4}$ et M.

9.- Procédé selon l'une des revendications 3, 6, 7 et 8, caractérisé en ce que l'extraction est effectuée à une température comprise entre 20°C et 280°C et, de préférence, entre 80 et 200°C.

10.- Procédé selon l'une des revendications 3, 6, 7, 8 et 9, caractérisé en ce que la durée de l'extraction est comprise entre 0,5 et 192 heures, et, de préférence, entre 1 et 120 heures.

11.- Procédé selon l'une des revendications 5 et 6, caractérisé en ce que, lors de l'extraction, la concentration de l'agent complexant est comprise en mole par litre, entre $\frac{M}{10}$ et 2 M, et, de préférence, entre $\frac{M}{5}$ et M.

12.- Procédé selon l'une des revendications 5, 6 et 11, caractérisé en ce que l'agent complexant est un sel de l'acide éthylènediamine-tétraacétique et en ce qu'une base est employée conjointement avec l'agent complexant à une concentration, en mole par litre, comprise entre $\frac{M}{10}$ et 2 M et, de préférence, entre $\frac{M}{5}$ et 1,5 M.

13.- Procédé selon l'une des revendications 5, 6, 11 et 12, caractérisé en ce que l'agent complexant est un sel de l'acide éthylène diaminetétraacétique et en ce que l'extraction est effectuée à une température comprise entre 40°C et 180°C et, de préférence, entre

60°C et 150°C.

14.- Procédé selon l'une des revendications 5, 6, 11, 12 et 13, caractérisé en ce que l'agent complexant est un sel de l'acide éthylènediamine tétraacétique et en ce que la durée de l'extraction est comprise entre 6 et 192 heures et, de préférence, entre 12 et 96 heures.

15.- Procédé selon l'une des revendications 5, 6 et 11, caractérisé en ce que l'agent complexant est un sel de l'acide diéthylène triamine pentaacétique et en ce qu'une base est employée conjointement avec l'agent complexant à une concentration, en mole par litre, comprise entre $\frac{M}{2}$ et 2 M et, de préférence, entre M et 1,5 M.

16.- Procédé selon l'une des revendications 5, 6, 11 et 15, caractérisé en ce que l'agent complexant est un sel de l'acide diéthylène triamine pentaacétique et en ce que l'extraction est effectuée à une température comprise entre 20°C et 125°C et, de préférence, entre 40°C et 110°C.

17.- Procédé selon l'une des revendications 5, 6, 11, 15 et 16, caractérisé en ce que l'agent complexant est un sel de l'acide diéthylène triamine pentaacétique et en ce que la durée de l'extraction est comprise entre 6 et 144 heures et, de préférence, entre 12 et 72 heures.

18.- Procédé selon l'une des revendications 1 à 17, caractérisé en ce que la zéolite ZK 5 est obtenue conditionnée, la zéolite de départ étant déjà conditionnée.

19.- La zéolite ZK 5 préparée par un procédé selon l'une des revendications 1 à 18.

20.- Application de la zéolite ZK 5 selon la revendication 19 à la séparation d'hydrocarbures aliphatiques saturés linéaires et d'hydrocarbures aliphatiques saturés ramifiés.

21.- Application de la zéolite ZK 5 selon la revendication 19 à la séparation d'hydrocarbures aliphati-

ques saturés linéaires et d'hydrocarbures cycliques saturés.

22.- Application de la zéolite ZK 5 selon la revendication 19 à la séparation d'hydrocarbures aliphatiques saturés linéaires et d'hydrocarbures aromatiques.

23.- Application de la zéolite ZK 5 selon la revendication 19 comme catalyseur de craquage d'hydrocarbures.

0096598
Numéro de la demande

Office européen des brevets

# RAPPORT DE RECHERCHE EUROPEENNE

EP 83 40 0482

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. 3) |
|---|---|---|---|
| D,A | JOURNAL OF THE CHEMICAL SOCIETY, section A, partie IV, 1970, pages 2735-2745, Londres, GB. R.M. BARRER et al.: "Hydrothermal chemistry of silicates. Part XV. Synthesis and nature of some salt-bearing aluminosilicates" * En entier, sauf page 2741, colonne de droite "Species N and O" - page 2744, colonne de droite, ligne 2; plus spécialement page 2737, colonne de droite "The structure of species P,Q,P', and Q' - page 2741 * |  | C 01 B 33/28<br>C 07 C 7/13<br>C 07 C 4/06<br>B 01 J 29/28 |
| A | JOURNAL OF THE CHEMICAL SOCIETY, 1953, pages 1466-1475, Londres, GB. R.M. BARRER et al.: "The hydrothermal chemistry of silicates. Part III. Reactions of analcite and leucite" * Page 1469, en bas "Reactions with halides" - page 1474, alinéa 3, hormis page 1471 "Reactions of Analoite with miscellaneous salts" et 1ère moitié page 1473* |  | DOMAINES TECHNIQUES RECHERCHES (Int. Cl. 3)<br>C 01 B 33/00 |
| A | JOURNAL OF THE CHEMICAL SOCIETY, partie I, 1948, pages 127-132, Londres, GB. R.M. BARRER: "Synthesis of a zeolitic mineral with chabazite-like sorptive properties" |  |  |
|  | --- -/- |  |  |

Le présent rapport de recherche a été établi pour toutes les revendications

| Lieu de la recherche | Date d'achèvement de la recherche | Examinateur |
|---|---|---|
| LA HAYE | 08-07-1983 | BREBION J.CH. |

CATEGORIE DES DOCUMENTS CITES

X : particulièrement pertinent à lui seul
Y : particulièrement pertinent en combinaison avec un autre document de la même categorie
A : arrière-plan technologique
O : divulgation non-écrite
P : document intercalaire
T : théorie ou principe à la base de l'invention
E : document de brevet antérieur, mais publié à la date de dépôt ou après cette date
D : cité dans la demande
L : cité pour d'autres raisons
& : membre de la même famille, document correspondant

OEB Form 1503 03 82

Office européen des brevets

**RAPPORT DE RECHERCHE EUROPEENNE**

0096598
Numéro de la demande

EP 83 40 0482



| DOCUMENTS CONSIDERES COMME PERTINENTS | | | |
|---|---|---|---|
| Catégorie | Citation du document avec indication, en cas de besoin, des parties pertinentes | Revendication concernée | CLASSEMENT DE LA DEMANDE (Int. Cl. 3) |
| D,A | US-A-2 413 134 (R.M. BARRER)<br>--- | | |
| A | JOURNAL OF THE CHEMICAL SOCIETY, partie I, 1948, pages 133-143, Londres, GB. R.M. BARRER et al.: "Sorptive and molecular-sieve properties of a new zeolitic mineral"<br>--- | | |
| A | FR-A-1 351 708 (SOCONY MOBIL OIL CO.)<br>--- | | |
| A | US-A-3 720 753 (H.E. ROBSON)<br>----- | | DOMAINES TECHNIQUES RECHERCHES (Int. Cl. 3) |

Le présent rapport de recherche a été établi pour toutes les revendications

| Lieu de la recherche | Date d'achèvement de la recherche | Examinateur |
|---|---|---|
| LA HAYE | 08-07-1983 | BREBION J.CH. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03.82